# NEW EUROPEAN PATENT SPECIFICATION

(11) **EP 0 069 423 B2**
(45) Date of publication and mention of the opposition decision: **22.04.1998**
(45) Mention of the grant of the patent: 27.04.1988
(21) Application number: 82200803.3
(22) Date of filing: 29.06.1982
(51) Int. Cl.: A61K 9/10

(54) **Composition for topical application to the body**
Mittel zur örtlichen Anwendung am Körper
Composition pour application topique au corps

(30) Priority: 02.07.1981 GB 8120471
(43) Date of publication of application: 12.01.1983
(73) Proprietor: YAMANOUCHI EUROPE B.V., 2350 AC Leiderdorp (NL)
(72) Inventor: Molenaar, Adrianus Pieter, NL-2625 AS Delft (NL)

(56) References cited:
- EP-A- 0 042 827
- EP-S- 65 929
- DE-A- 264 435
- DE-A- 264 435
- DE-A- 408 663
- FR-A- 2 258 165
- NL-A- 267 783
- NL-A- 271 464
- US-A- 246 261
- US-A- 435 324
- US-A- 3 758 686
- US-A- 4 244 942
- G. Rajka & Col. J Int. Med. Res.14 (1986) p.85-90
- P. Bazey, Extrait de Tribune Medicale p. 190 (1986)
- Handbuch der Kosmetika und Riechstoffe - Janistin (1973) p. 558-560
- Remington's Pharmaceutical Sciences (1975) 15th Ed. p. 334-336, 1453
- L. Gip "Therapeutic Research 34 (1985)" p. 813-817
- F.Gstirner, Grundstoffe u. Verfahren der Arzneiberatung, F.Enke Verlag Stuttgart, 1960, S.696
- H.Janystin, Taschenbuch der modernen Parfümerie u. Kosmetik, Wissenschaftliche Verlagsgesellschaft mbH, Stuttgart, 4. Auflage, 1974, S.508
- Europ. Pharmacopoeia, 2.Edit., Maisonneuve S.A., 1981, Part II, pp. 132, 132-2, 132-3
- United States Pharmacopeia, Twenty-First Revision 1980, pp. 1135-1137
- "Cosmetics, Science and Techn.", 2.Edition, ed. M.S.Balsam and E.Sagarin, J.Wiley and Sons Inc., 1972, vol. 1, pp.44-45, 54-55, vol. 3, pp.573-629
- "Formulary of Cosmetic Preparations" M.Ash and I.Ash, G.Goodwin Ltd., 1977, pp. 266-269, 280-281
- "Dermatologic Formulary", ed. F.Pascher, Hoeber Harper, 1957, pp. 40-41, 58-61
- "Topicals", AmercholCorporation, 1978, pp. II-3, II-7, II-14, IV-1, IV-3
- "The Pharmaceutical Codex", 11.Edition, Pharmaceutical Society of Great Britain, 1979, pp. 154-155
- P.Becher, "The Effect of the Nature of the EMulsifying Agent on emulsion inversion", J.Soc. Cosmetic Chemists, 1958, 9, 141-148
- B.W.Barry, "The control of oil-in-water emulsion consistency using mixed emulsifiers", J.Pharm.Pharmac., 1969, 21, 533-540
- K.Shinoda and H.Saito, J.of Colloid and Interface Science, 1968, 26, pp.70-74, "The effect of Temperature of the Phase Equilibira ...
- F.A.J.Talman, P.J.Davies and E.M.Rowan, J.Pharm.Pharmac. 1967, 19, 417-425, "The rheology of some oil-in-water emulsions stabilised by condensed complex films"
- F.A.J.Talman, P.J.Davies and E.M.Rowan, J.Pharm.Pharmacl, 1968, 20, 513-520, "The effect of the concentration of the water-soluble component on ...
- Croda Chemicals Ltd. 1979 (extracts), with covering letter, dated 17th July, 1979, from Croda Chem. Ldt., "Cosmetic/pharmac. formulary"
- J.T.DaviesL Proc. Int. Cong. Surface Active, 3rd, Cologne 1961, Untergruppe B/IV/2, Nr.83, Univ. of Cambridge (UK), pp. 585-594
- Martindale, "The Extra Pharmacopoeia", ed. A.Wade, Pharmaceutical Press, 1977, p. 1049
- "Handbuch der Kosmetika u. Reichstoffe", H.Janistyn, Dr.A.Huethig Verlag, 1973, pp. 558-560
- "Harry's Cosmeticology", 6.edition, R.G.Harry, ed. J.B.Wilkinson et al, Leonard Hill Books, 1975, pp 53-54, 691-726
- Summary (in Engl.) of response filed on 9 March 87 by patentees Gist-Brocades in Finnish Patent Application no. 822331

## Description

This invention is concerned with pharmaceutical topical formulations.

It is well known that *inter alia* therapeutic agents can be applied to the skin by means of for example, ointments, creams and lotions. Various skin afflictions can react in different ways to the composition of the vehicle through which the therapeutic agent is administered to the skin. For those afflictions which cause the skin to become dry and flaky the use of an ointment is generally preferred. An ointment generally contains more oil (fatty components) than water; sometimes an ointment contains only fatty constituents. On the other hand, there are skin afflictions in which occlusion of the skin, in order to avoid drying up, is not desirable and in such cases it is better to use a cream containing more water than oil. Ointments have the disadvantage of being sticky and greasy to the touch, and are not easily removed by ordinary washing of the skin or clothing which comes into contact with the ointment. Lotions are generally non-visous and consequently therapeutic agents contained therein are only in contact with the skin for a relatively brief period of time as lotions run off the skin.

To obtain a stable vehicle for ointments containing water and creams an emulsifying agent (a surfactant) is used in their preparation. There are emulsifiers with a lipophilic character (to 'disperse' water in oil, i.e. a W/O emulsifers) or with a hydrophilic character (to 'disperse' oil in water, i.e. an O/W emulsifiers). In the case of an ointment (more oil than water) usually a W/O emulsifier is used; the ointment consists of a continuous oil phase in which small water particles are present. In the case of a cream (more water than oil) an O/W emulsifier is used; the cream consists of a continuous water phase in which small oil particles are present. To ascertain whether formulations are ointments or creams coloured compounds can be used which dissolve either in the oil-phase or the water-phase, and by means of a microscope a distinction can be made.

"New Zealand patent No. 187103 concerns the specific problem of preventing the oxidation of dithranol, when incorporated in the fat phase of a stiff emulsion to be used for treating psoriasis. This problem is solved by adding an anti-oxidant and an acid to the agueous phase of an o/w emulsion wherein dithranol is in the fat phase. The claims of New Zealand patent No. 187103 broadly cover o/w emulsions containing from 20 to 60% of petroleum jelly and from 5 to 20% of an emulsifier."

As mentioned heretofore in the manufacture of a cream (more water than oil) an oil-in-water (O/W) emulsifier is used. It has now been found that stable compositions in the form of 'fatty-creams', which contain more fatty compounds than water, can be obtained when an O/W non-ionic emulsifying agent (or surfactant) of hydrophilic character is used; this is new and quite unexpected in the therapeutic field.

The surfactant (or emulsifer) should preferably have a high HLB (Hydrophilic-Lipophilic Balance) number. This term has been defined in the article by Balsam *et al*, Cosmetics, Science and Technology, Vol. III, pages 583-596. Advantageously the HLB number should be 14 or above.

Thus, the present invention relates to a topical composition in the form of fatty creams for application to the body, which comprises a fatty base, water and at least one non-ionic surfactant, the composition containing from 60 to 80% by weight of fatty material(s), from 1.5 to 5% by weight of surfactant(s); the surfactant(s) having a HLB (Hydrophilic-Lipophilic Balance) number of 14 or above, and a therapeutic agent with the exception of dithranol and its derivatives. Preferably, the composition contains 60 to 70% by weight of fatty material.

The 'fatty-creams' of the present invention can be prepared by the application of conventional methods for the manufacture of ointments and creams. Microscopic examination of them shows sometimes a picture of a complex emulsion, and sometimes an oil in water emulsion, depending on the intensity of the mixing procedure. In case of a complex emulsion the continuous phase is in some parts of the emulsion the oily components and in other parts the continuous phase is water. The fatty-creams have an occlusive action when applied to skin due to the high oil content; the skin becomes moist and therefore better penetrable by the therapeutic agent employed therein. Moreover the fatty-creams, in contrast with ointments, are non-greasy and readily removable from the skin or material which come into contact with them.

The fatty materials which are incorporated in the fatty-creams of the present invention are those commonly used in the preparation of ointments and creams. Preferably a mixture of fatty compounds (solid, semi-solid and/or liquid at ambient temperature) is used in the preparation of the fatty-creams. The fatty compounds may be, for example, waxes (e.g. white soft paraffin), liquid paraffin, fatty alcohols and esters (e.g. cetyl stearyl alcohol, myristyl alcohol and glycerin monostearate), vegetable oils (e.g. cottonseed, coconut. soybean or peanut oil), mineral oils or liquid silicones. Preferably a combination of cetyl stearyl alcohol, liquid paraffin and white soft paraffin is employed.

The amount of hydrophilic non-ionic surfactant (liquid or solid) used in the preparation of the fatty-creams of the present invention is preferably from 1.5 to 3.5% (advantageously 3%) by weight of the final composition. The surfactant is preferably cetomacrogol 1000 (i.e. polyethylene glycol (1000) monoceryl ether); other surfactants which may be used are polysorbate 60 (i.e. polyoxyethylene (20) sorbitan monostearate), and polysorbate 80 or Tween 80 (i.e. polyoxyethylene (20) sorbitan monooleate). Advantageously only one surfactant is employed.

The amount of water (preferably de-ionised) used in the preparation of the fatty-creams of the present invention may range from 20% up to 35% by weight of the final composition. Advantageously about 30% of water is used.

The topically-active therapeutic agent(s) with the exception of dithranol and its derivatives incorporated in the fatty-creams of the present invention may be a steroid (for example an anti-inflammatory steroid), an antibiotic or a chemotherapeutic agent, or a combination of such therapeutically useful agents. The therapeutic agent may be, for example, hydrocortisone-17α-butyrate, hydrocortisone, triamcinolone acetonide, salicylic acid and derivatives thereof, tar products, sulphur compounds, iodine compounds, nicotinic acid and derivatives thereof, hexachlorophene or retinoic acid. The amount of therapeutic agent, which may be soluble in the aqueous- or oil-phase of the fatty-cream, will be that suitable for the application intended.

When the therapeutic agent (e.g. hydrocortisone-17α-butyrate) is soluble in the aqueous-phase of the fatty cream, a better activity can be obtained than in a normal cream or ointment as the therapeutic agent is dissolved in a smaller volume of water, leading to a higher concentration of the therapeutic agent.

It may be useful to include in the fatty-reams of the present invention a buffering agent to maintain a desired pH value. The buffering agent may be, for example, a combination of (i) citric acid and sodium citrate, (ii) phosphoric acid and sodium phosphate, or (iii) lactic acid and sodium lactate, as appropriate for the desired pH. Where the therapeutic agent is hydrocortisone-17α-butyrate a slightly acidic environment (e.g. pH 3.5 to 4.5) is needed in order to prevent hydrolysis of the butyrate; a combination of citric acid and sodium citrate is particularly useful for the purpose.

The fatty-creams of the present invention may also incorporate-as is common practice in ointments and cream-a preservative to prevent, for example, bacterial attack. Suitable preservatives are methyl hydroxybenzoate, chlorocresol, sorbic acid and benzoic acid.

As mentioned heretofore, the fatty-creams of the present invention may be prepared by conventional means. A preferred method involves mixing the 'fatty' components (e.g. cetyl stearyl alcohol, liquid paraffin and white soil paraffin) with the hydrophilic non-ionic surfactant (preferably cetomacrogol 1000) and heating the mixture at, for example, 70-80°C. Separately in a suitable ointment-mixer water (preferably de-ionised), buffering agent and preservative are mixed and heated (e.g. at 70-80°C) until a solution is obtained. A small portion of the resulting solution is separated and cooled to ambient temperature, and then the therapeutic agent is added to it. The liquid fatty composition at elevated temperature (e.g. 70-80°C) is added to the larger portion of the aqueous solution and then, after vigorous stirring under reduced pressure (to avoid air bubbles in the final fatty-cream), the aqueous solution or suspension containing the therapeutic agent is added to it at normal pressure. After vigorously mixing the mass at about ambient temperature for an appropriate length of time-once again under reduced pressure-a fatty-cream is obtained.

The temperature of the final mixing of the components of the fatty-cream and the agitation involved can affect the form of the emulsion (oil-in-water and/or water-in-oil) obtained. Preferably it is effected at 20-25°C with vigorous agitation.

The fatty-creams of the present invention so obtained preferably comprise 'oil' globules with a mean diameter of 1-2 µm (maximum 5 µm). As a result of the high fat-content, an aqueous phase is usually hardly perceptible. Possibly the water is attached as a thin film to the surface of the globules, still forming the continuous phase.

The fatty smooth creams of the present invention can be extremely stable under normal conditions, especially when they are emulsions containing mostly oil particles of very small mean diameter, e.g. 1-2 µm.

The following Examples illustrate the preparation of fatty-creams according to the present invention.

### Example 1

50 kg of a fatty-cream was prepared as follows:-
3000 g of cetyl stearyl alcohol, 1500 g of cetomacrogol 1000, 9000 g of liquid paraffin and 21000 g of white soft paraffin were mixed together and heated to 70-80°C.
   In a suitable ointment-mixer (e.g. a 50 kg Unimix, manuf. Heagen and Rinau, Bremen, Federal Republic of Germany) 60 g of sodium citrate. 90 g of citric acid and 100 g of methyl hydroxybenzoate were added to 15500 g of deionised water.-While stirring, this mixture was heated to 70-80°C and kept at this temperature until the components were dissolved. 2000 g of the resulting solution were separated and cooled to room temperature.
   The prepared liquid fatty composition was added at a temperature of 70-80°C to the remaining solution. After vigorous mixing, under reduced pressure (50 mm Hg), the mass was gradually cooled down to 20-25°C.
   50 g of hydrocortisone-17α-butyrate was evenly suspended in the separated portion of the aqueous solution (e.g. in an Ultra-Turrax mixer, manuf. Janke and Kunkei, West-Germany). After releasing the vacuum in the ointment-mixer using filtered air, the suspension containing hydrocortisone-17α-butyrate was added to the cream. After closure of the mixer and vigorous mixing of the contents under reduced pressure (50 mm Hg) for at least half an hour, the cream was homogenized. After releasing the vacuum, the fatty-cream (50 kg) was transferred into a container, from which it can be filled into tubes and jars.
   The fatty-cream on examination under a microscope comprised oil-globules with a mean diameter of 1-2 µm, the larger ones being up to 5 µm.

### Example 2

The procedure of Example 1 to obtain a fatty-cream was followed but replacing the 50 g of hydrocortisone-17α-butyrate by 500 g of hydrocortisone and using 15050 g of de-ionised water instead of 15500 g.

### Example 3

The procedure of Example 1 to obtain a fatty-cream was followed but replacing the 50 g of hydrocortisone-17α-butyrate by 50 g of triamcinolone acetonide.

### Example 4

The procedure of Example 1 was followed except that the hydrocortisone-17α-butyrate (viz. the therapeutic agent) was omitted; consequently the step of separation of 2000 g of the prepared aqueous solution was not effected.

A fatty-cream base, which can be used as a pharmaceutical or therapeutical aid, was obtained.

### Examples 5 to 8

The procedures of Examples 1 to 4 were followed but replacing the cetyl stearyl alcohol by glyceryl monostearate.

### Examples 9 to 12

The procedures of Examples 1 to 4 were followed but replacing the cetomacrogol 1000 by polysorbate 80.

### Examples 13 to 16

The procedures of Examples 1 to 4 were followed but replacing the liquid paraffin by a vegetable oil selected from cottonseed, refined coconut and soybean oils.

### Examples 17 to 20

The procedures of Examples 1 to 4 were followed but replacing part or all the white soft paraffin by a corresponding amount of hardened peanut oil.

### Examples 21 to 24

The procedures of Examples 1 to 4 were followed but replacing the citric acid and sodium citrate by other buffering systems, viz. phosphoric acid and sodium phosphate, modification of the ratio of both giving the desired pH.

### Examples 25 to 28

The procedures of Examples 1 to 4 were followed except that no buffering agent (viz. citric acid and sodium citrate) was added.

### Examples 29 to 32

The procedures of Examples 1 to 4 were followed with the methyl hydroxybenzoate replaced by another preservative selected from chlorocresol, sorbic acid and benzoic acid.

All the products of Examples 5 to 32 were fatty-creams similar to that obtained in Example 1.

## Claims (Claims for the following Contracting State(s): BE, CH, DE, FR, GB, IT, LI, LU, NL, SE)

1. A topical composition in the form of a fatty-cream for application to the body which comprises a fatty base, water and at least one non-ionic surfactant, the composition containing from 60 to 80% by weight of fatty material(s), from 1.5 to 5% by weight of surfactant, the surfactant(s) having a HLB (Hydrophilic-Lipophilic Balance) number of 14 or above, and a therapeutic agent with the exception of dithranol and its derivatives

2. A topical composition according to claim 1 in which the amount of fatty material(s) is from 60 to 70% by weight of the composition.

3. A topical composition according to claim 1 or 2 in which the fatty materials included in the composition are cetyl stearyl alcohol, liquid paraffin and white soft paraffin.

4. A composition according to any one of the preceding claims in which the amount of surfactant is from 1.5 to 3.5% by weight of the composition.

5. A composition according to any one of the preceding claims in which the hydrophilic non-ionic surfactant is cetomacrogol 1000.

6. A composition according to any one of the preceding claims in which the amount of water present is from 20% up to 35% by weight of the composition.

7. A topical composition according to claim 6 in which the amount of water is about 30% by weight.

8. A topical composition according to any one of the preceding claims in which therapeutic agent is a steroid.

9. A topical composition according to claim 8 in which the steroid is hydrocortisone-17-alpha-butyrate.

10. A topical composition according to any one of the preceding claims which includes a buffering agent to maintain a desired pH value in the composition.

11. A topical composition according to claim 10 in which the buffering agent is a combination of citric acid and sodium citrate.

12. A topical composition according to any one of the preceding claims which includes a preservative.

## Claims (Claims for the following Contracting State(s): AT)

1. Process for the preparation of a topical composition in the form of a fatty cream for application to the body, characterized in that it comprises a fatty base, water, at least one hydrophilic non-ionic surfactant having a HLB (Hydrophilic-Lipophilic Balance) number of 14 or above, from 60 to 80% by weight of fatty material(s) and from 1.5 to 5% by weight of surfactant, and a therapeutic agent with the exception of dithranol and its derivatives

2. Process according to claim 1 in which the amount of fatty material(s) is from 60 to 70% by weight of the composition.

3. Process according to claim 1 or 2 in which the fatty materials included in the composition are cetyl stearyl alcohol, liquid paraffin and white soft paraffin.

4. Process according to claim 1, 2 or 3 in which the amount of surfactant is from 1.5 to 3.5% by weight of the composition.

5. Process according to any one of the preceding claims in which the hydrophilic non-ionic surfactant is cetomacrogol 1000.

6. Process according to any one of the preceding claims in which the amount of water present is from 20% up to 35% by weight of the composition.

7. Process according to claim 6 in which the amount of water is about 30% by weight.

8. Process according to any one of claims 1 to 7 which includes, as, the therapeutic agent, a steroid.

9. Process according to claim 8 in which the steroid is hydrocortisone-17-alpha-butyrate.

10. Process according to any one of the preceding claims which includes a buffering agent to maintain a desired pH value in the composition.

11. Process according to claim 10 in which the buffering agent is a combination of citric acid and sodium citrate.

12. Process according to any one of the preceding claims which includes a preservative.

## Patentansprüche (Patentansprüche für folgende(n) Vertragsstaat(en): BE, CH, DE, FR, GB, IT, LI, LU, NL, SE)

1. Mittel zur örtlichen Anwendung am Körper in Form einer fetthaltigen Creme, das eine fetthaltige Grundlage, Wasser und mindestens eine nicht ionogene oberflächenaktive Substanz enthalt, wobei das Mittel 60 bis 80 Gew.-% Fettstoff bzw. Fettstoffe, 1,5 bis 5 Gew.-% oberflächenaktive Substanz, wobei die oberflächenaktive Substanz bzw. die oberflächenaktiven Substanzen einen HLB-(Hydrophilic-Lipophilic Balance)-Wert von 14 oder darüber hat bzw. haben, sowie einen therapeutischen Wirkstoff ausgenommen Dithranol und Derivate davon enthält.

2. Mittel zur örtlichen Anwendung nach Anspruch 1, worin die Menge an Fettstoff bzw. Fettstoffen 60 bis 70 Gew.-% des Mittels beträgt.

3. Mittel zur örtlichen Anwendung nach Anspruch 1 oder 2, worin die Fettstoffe, die in das Mittel eingeschlossen sind, Cetylstearylalkohol, flüssiges Paraffin und weisses Weichparaffin sind.

4. Mittel nach einem der vorangehenden Ansprüche, worin die Menge an oberflächenaktiver Substanz 1,5 bis 3,5 Gew.-% des Mittels beträgt.

5. Mittel nach einem der vorangehenden Ansprüche, worin die hydrophile nicht ionogene oberflächenaktive Substanz Cetomacrogol 1000 ist.

6. Mittel nach einem der vorangehenden Ansprüche, worin die vorhandene Wassermenge 20 bis zu 35 Gew.-% des Mittels beträgt.

7. Mittel zur örtlichen Anwendung nach Anspruch 6, worin die Wassermenge ca. 30 Gew.-% beträgt.

8. Mittel zur örtlichen Anwendung nach einem der vorangehenden Ansprüche, worin der therapeutische Wirkstoff ein Steroid ist.

9. Mittel zur örtlichen Anwendung nach Anspruch 8, worin das Steroid Hydrocortison-17-alpha-butyrat ist.

10. Mittel zur örtlichen Anwendung nach einem der vorangehenden Ansprüche, das ein Puffermittel einschliesst, um in dem Mittel einen gewünschten pH-Wert aufrechtzuerhalten.

11. Mittel zur örtlichen Anwendung nach Anspruch 10, worin das Puffermittel eine Kombination von Zitronensäure und Natriumcitrat ist.

12. Mittel zur örtlichen Anwendung nach einem der vorangehenden Ansprüche das ein Konservierungsmittel enthält.

## Patentansprüche (Patentansprüche für folgende(n) Vertragsstaat(en): AT)

1. Verfahren zur Herstellung eines Mittels zur örtlichen Anwendung am Körper in Form einer fetthaltigen Creme, dadurch gekennzeichnet, dass es eine fetthaltige Grundlage, Wasser, mindestens eine hydrophile nicht ionogene oberflächenaktive Substanz mit einem HLB-(Hydrophilic-Lipophilic Balance)-Wert von 14 oder darüber, 60 bis 80 Gew.-% Fettstoff bzw. Fettstoffe und 1,5 bis 5 Gew.-% oberflächenaktive Substanz sowie einen therapeutischen Wirkstoff ausgenommen Ditharanol und Derivate davon enthält.

2. Verfahren nach Anspruch 1, worin die Menge an Fettstoff bzw. Fettstoffen 60 bis 70 Gew.-% des Mittels beträgt.

3. Verfahren nach Anspruch 1 oder 2, worin die Fettstoffe, die in das Mittel eingeschlossen sind, Cetylstearylalkohol, flüssiges Paraffin und weisses Weichparaffin sind.

4. Verfahren nach Anspruch 1, 2 oder 3, worin die Menge an oberflächenaktiver Substanz 1,5 bis 3,5 Gew.-% des Mittels beträgt.

5. Verfahren nach einem der vorangehenden Ansprüche, worin die hydrophile nicht ionogene oberflächenaktive Substanz Cetomacrogol 1000 ist.

6. Verfahren nach einem der vorangehenden Ansprüche, worin die vorhandene Wassermenge 20 bis zu 35 Gew.-% des Mittels beträgt.

7. Verfahren nach Anspruch 6, worin die Wassermenge ca. 30 Gew.-% beträgt.

8. Verfahren nach einem der Ansprüche 1 bis 7, das als therapeutischen Wirkstoff ein Steroid einschliesst.

9. Verfahren nach Anspruch 8, worin das Steroid Hydrocortison-17-alpha-butyrat ist.

10. Verfahren nach einem der vorangehenden Ansprüche, das ein Puffermittel einschliesst, um in dem Mittel einen gewünschten pH-Wert aufrechtzuerhalten.

11. Verfahren nach Anspruch 10, worin das Puffermittel eine Kombination von Zitronensäure und Natriumcitrat ist.

12. Verfahren nach einem der vorangehenden Ansprüche, das ein Konservierungsmittel eintschliesst.

## Revendications (Revendications pour l'(les) Etat(s) contractant(s) suivant(s): BE, CH, DE, FR, GB, IT, LI, LU, NL, SE)

1. Composition topique sous la forme d'une crème grasse pour l'application sur le corps, qui comprend une base grasse, de l'eau et au moins un tensioactif non ionique, la composition contenant 60 à 80% en poids de matière(s) grasse(s), 1,5 à 5% en poids de tensioactif(s), le ou les tensioactifs ayant un indice BHL (bilan hydrophile-lipophile) de 14 ou davantage, et un agent thérapeutique à l'exception de dithranol et ses dérivés

2. Composition topique suivant la revendication 1, dans laquelle la quantité de matière(s) grasse(s) est de 60 à 70% du poids de la composition.

3. Composition topique suivant la revendication 1 ou 2, dans laquelle les matières grasses comprises dans la composition sont l'alcool cétostéarylique, la paraffine liquide et la paraffine blanche molle.

4. Composition suivant l'une quelconque des revendications précédentes, dans laquelle la quantité de tensioactif est de 1,5 à 3.5% du poids de la composition.

5. Composition suivant l'une quelconque des revendications précédentes, dans laquelle le tensioactif non ionique hydrophile est le cétomacrogol 1000.

6. Composition suivant l'une quelconque des revendications précédentes, dans laquelle la quantité d'eau présente est de 20% jusqu'à 35% du poids de la composition.

7. Composition topique suivant la revendication 6, dans laquelle la quantité d'eau est d'environ 30% en poids.

8. Composition topique suivant l'une quelconque des revendications précédentes, dans laquelle l'agent thérapeutique est un stéroïde.

9. Composition topique suivant la revendication 8, dans laquelle le stéroïde est le 17α-butyrate d'hydrocortisone.

10. Composition topique suivant l'une quelconque des revendications précédentes, qui comprend un agent tampon pour maintenir une valeur souhaitée du pH dans la composition.

11. Composition topique suivant la revendication 10, dans laquelle l'agent tampon est une combinaison d'acide citrique et de citrate de sodium.

12. Composition topique suivant l'une quelconque des revendications précédentes, qui comprend un conservateur.

## Revendications (Revendications pour l'(les) Etat(s) contractant(s) suivant(s): AT)

1. Procédé de préparation d'une composition topique sous la forme d'une crème grasse pour l'application sur le corps, caractérisé en ce qu'il comprend une base grasse, de l'eau, au moins un tensioactif non ionique hydrophile ayant un indice BHL (bilan hydrophile-lipophile) de 14 ou davantage, 60 à 80% en poids de matière(s) grasse(s) et 1,5 à 5% en poids de fensioactif(s), et un agent thérapeutique à l'exception de dithranol et ses dérivés

2. Procédé suivant la revendication 1, dans lequel la quantité de matière(s) grasse(s) est de 60 à 70% du poids de la composition.

3. Procédé suivant la revendication 1 ou 2, dans lequel les matières grasses comprises dans la composition sont l'alcool cétostéarylique, la paraffine liquide et la paraffine blanche molle.

4. Procédé suivant la revendication 1, 2 ou 3, dans lequel la quantité de tensioactif est de 1,5 à 3,5% du poids de la composition.

5. Procédé suivant l'une quelconque des revendications précédentes, dans lequel le tensioactif non ionique hydrophile est le cétomacrogol 1000.

6. Procédé suivant l'une quelconque des revendications précédentes, dans lequel la quantité d'eau présente est de 20% jusqu'à 35% du poids de la composition.

7. Procédé suivant la revendication 6, dans lequel la quantité d'eau est d'environ 30% en poids.

8. Procédé suivant l'une quelconque des revendications 1 à 7, qui met en jeu un stéroïde comme agent thérapeutique.

9. Procédé suivant la revendication 8, dans lequel le stéroïde est le 17α-buryrate d'hydrocortisone.

10. Procédé suivant l'une quelconque des revendications précédentes, qui met en jeu un agent tampon pour maintenir une valeur souhaitée du pH dans la composition.

11. Procédé suivant la revendication 10, dans lequel l'agent tampon est une combinaison d'acide citrique et de citrate de sodium.

12. Procédé suivant l'une quelconque des revendications précédentes, qui met en jeu un conservateur.
